# EUROPÄISCHE PATENTSCHRIFT

(11) **EP 0 561 854 B1**
(45) Veröffentlichungstag und Bekanntmachung des Hinweises auf die Patenterteilung: **17.07.1996**
(21) Anmeldenummer: 92900042.0
(22) Anmeldetag: 05.12.1991
(51) Int. Cl.: C09J 7/02, A61B 5/04

(54) **ELEKTRISCH LEITFÄHIGE TRANSPARENTE HAFTKLEBEFILME, VERFAHREN ZU IHRER HERSTELLUNG UND VERWENDUNG ZUR HERSTELLUNG BIOMEDIZINISCHER ELEKTRODEN**
ELECTROCONDUCTIVE, TRANSPARENT PRESSURE-SENSITIVE ADHESIVE FILMS, PROCESS FOR PRODUCING THE SAME AND THEIR USE TO PRODUCE BIOMEDICAL ELECTRODES
PELLICULES AUTO-ADHESIVES TRANSPARENTES ELECTROCONDUCTRICES, LEUR PROCEDE DE PRODUCTION ET LEUR UTILISATION POUR PRODUIRE DES ELECTRODES BIOMEDICALES

(30) Priorität: 13.12.1990 DE 4039780
(43) Veröffentlichungstag der Anmeldung: 29.09.1993
(73) Patentinhaber: Lohmann GmbH & Co. KG, D-56567 Neuwied (DE)
(72) Erfinder: CZECH, Zbigniew, D-5400 Koblenz 1 (DE)
(74) Vertreter: Flaccus, Rolf-Dieter, Dr.
(86) Internationale Anmeldenummer: EP9102318
(87) Internationale Veröffentlichungsnummer: WO9210553

(56) Entgegenhaltungen:
- EP-A- 0 177 139
- EP-A- 0 322 098
- US-A- 4 273 135
- US-A- 4 391 278
- US-A- 4 777 954

## Beschreibung

Die Erfindung betrifft elektrisch leitfähige transparente Haftklebefilme mit einer elektrischen Leitfähigkeit oberhalb von 10⁻⁵ S, die sich zur Herstellung biomedizinischer Elektroden eignen.

Die gängige Standardausführung solcher biomedizinischer Elektroden enthält in einem aus Schaumstoffmitteln ausgestanzten Loch ein elektrisch leitfähiges Gel, dessen Lagerfähigkeit wegen mit der Lagerungsdauer zunehmenden Austrocknung begrenzt ist. Ein weiterer Nachteil dieser Austrocknung ist, daß die Haut nach Gebrauch der Elektrode von Gelrückständen gereinigt werden muß bzw., daß es bei nicht sorgfältig vorgenommener Entfernung der Gelückstände zu Verschmutzungen der Kleidung des Patienten kommen kann.

Es wurden daher Bemühungen unternommen, diese Nachteile durch Bereitstellen einer elektrisch leitfähig eingestellten lagerbeständigen Haftklebebeschichtung zu beseitigen, bzw. wesentlich zu mindern.

Übliche Methoden zur Erzielung einer elektrischen Leitfähigkeit, beispielsweise Einbau von Graphitpulver, sind nicht anwendbar, da der Haftklebefilm transparent bleiben muß.

Nach DE-OS 1594137 sind haftklebende leitfähige Streifen bekannt, die filmbildende ionisierte organische Polymersalze enthalten. Die Polymere selbst sind nur wenig in Wasser löslich, werden aber durch die eingebauten, zur Salzbildung befähigten funktionellen Gruppen hydrophil und ionogen gemacht. Als geeignete Beispiele sind die Natriumsalze von sulfoniertem Styrol und Polybenzylsulfonat zu nennen. Eine weitere bevorzugte Gruppe sind die Salze von quaternierten Polymerisaten aus Dimethylaminoethyl-Methacrylat oder dessen Mischpolymerisate mit Butylacrylat und 4-Vinylpyridin, das mit Methylbromid oder Allylchlorid zu 95 bis 100 % quaterniert worden ist.

Nach der EP-A 0263586 werden zur Herstellung von elektrisch leitfähigen Haftklebefilmen leitfähige Haftklebemassen aus einem Wasserstoffdonator-Monomer (z.B. Acrylsäure) und einem Wasserstoffakzeptor-Monomer (z.B N-vinylpyrrolidon) verwendet.

Als Polymerisationsmedium dient ein Gemisch aus Wasser und Glycerin. Dem Copolymerisat wird noch ein wasserlösliches Salz und als Vernetzer bifunktionelles (Meth-)acrylat zugesetzt.

In der EP-A 0322098 ist eine Herstellung von elektrisch leitfähigen Haftklebefilmen beschrieben, bei der eine hydrophile Haftklebemasse auf Basis von N-Vinyllactam in Mischung mit einem Weichmacher und vernetzt mit mehrfunktionellen ethylenisch ungesättigten Derivaten verwendet wird.

Bei der Verwendung zur Herstellung von biomedizinischen Elektroden weisen diese bekannten Haftklebefilme jedoch diverse Mängel auf, die ihre praktische Anwendung einschränken.

Ihr Kohäsionsvermögen ist infolge ihrer Empfindlichkeit gegenüber Hautfeuchtigkeit gemindert.

Nach mehrstündigem Kontakt mit der Haut verbleiben beim Abziehen Klebstoffreste auf der Haut, wodurch die Wiederverwendung von derartigen mit diesen elektrisch leitfähigen Haftklebefilmen ausgerüsteten biomedizinischen Elektroden unmöglich ist.

Der vorliegenden Erfindung liegt daher die Aufgabe zugrunde, elektrisch leitfähige Haftklebefilme herzustellen, die exzellente Feuchteresistenz und stabile innere Festigkeit aufweisen und sich zur Herstellung von wiederverwendbaren biomedizinischen Elektroden bestens eignen.

Das der Erfindung zugrunde liegende Problem wird überraschend gelöst durch elektrisch leitfähige transparente Haftklebefilme mit einer elektrischen Leitfähigkeit oberhalb von 10⁻⁵ S, welche bestehen aus 100 Gewichtsteilen eines carboxylgruppenhaltigen Copolymerisats auf Acrylatbasis, 50 bis 150 Gewichtsteilen eines wasserlöslichen Amins, 50 bis 250 Gewichtsteilen eines Polyoxyalkylens mit einer Molekularmasse unter 1000 und/ oder eines Polyols und/oder dessen Derivaten, 50 bis 200 Gewichtsteilen einer Elektrolytlösung und 0,1 bis 6 Gewichtsteilen eines Vernetzungsmittels.

Die bevorzugten carboxylgruppenhaltigen Copolymerisate auf Acrylatbasis werden vorzugsweise durch eine radikalische Lösemittelpolymerisation von
a) 40 bis 80 Gew.-% Alkyl(meth)acrylaten mit 4 bis 12 C-Atomen im Alkylrest,
b) 10 bis 30 Gew.-% hydroxylgruppenhaltigen (Meth)acrylaten
c) 5 bis 30 Gew.-% einfach ungesättigten Carbonsäuren
d) 0,5 bis 20 Gew.-% von Salzen von ungesättigten organischen Sulfonsäuren,
e) 0,1 bis 5 Gew.-% von Salzen von carboxylgruppenhaltigen N-substituierten (Meth)acrylamidderivaten

### synthetisiert.

Als Alkyl(meth)acrylate mit 4 bis 12 C-Atomen im Alkylrest sind Butyl-, Pentyl-, Hexyl-, Heptyl-, Octyl-, 2-Ethylhexyl-, Isooctyl-, 2-Methylheptyl-, Nonyl-, Isononyl-, Decyl oder Dodecyl(meth)acrylat bevorzugt.

Als hydroxylgruppenhaltige (Meth)acrylate werden die Hydroxyalkyl(meth)acrylate bevorzugt, insbesondere 2-Hydroxyethyl(meth)acrylat, 2-Hydroxypropyl(meth)acrylat oder 4-Hydroxybutyl(meth)acrylat, die allein oder im Gemisch untereinander eingesetzt werden können.

Bevorzugte Vinylcarbonsäuren, die im fertigen Polymerisat aktive Vernetzungszentren bilden, sind (Meth)acrylsäure, β-Acryloyloxypropionsäure, Vinylessigsäure, Aconitsäure, Trichloracrylsäure, Dimethylacrylsäure, Crotonsäure, Fumarsäure oder Itaconsäure, insbesondere bevorzugt sind (Meth)acrylsäure und β-Acryloyloxypropionsäure.

Die Salze der ungesättigten organischen Sulfonsäuren, die zur Herstellung des carboxylgruppenhaltigen Copolymerisats auf Acrylatbasis eingesetzt werden, sind bevorzugt Metallsalze, insbesondere Alkalimetall- und/oder Ammoniumsalze, wobei die Salze der Vinylsulfonsäure, 2-Methylprop-1-en-3-sulfonsäure, Vinylbenzylsulonsäure und der 2-Acrylamido-2-methylpropansulfonsäure bevorzugt sind. Als Metallsalze sind insbesondere die Lithium-, Natrium- und/oder Kaliumsalze geeignet.

Als bekannte carboxylgruppenhaltige N-substituierte (Meth)acrylamidderivate kommen bevorzugt N-substituierte (Meth)acrylamidderivate der allgemeinen Formel 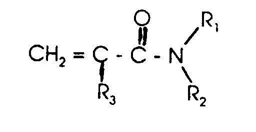 zum Einsatz, in der R₁ ein Wasserstoffatom, eine Alkyl-, Aryl-, Arylalkyl-, Alkylaryl-, Alkoxyalkyl-, Alkoxyaryl-, Acetylalkyl- oder Acetylalkoxyalkylgruppe, R₂ eine Carboxyalkyl- oder Carboxylarylgruppe, R₃ ein Wasserstoffatom oder eine Methylgruppe sein können. Als Salze dieser Verbindungen können vor allem die Alkali- oder Ammoniumsalze in Frage.

Das carboxylgruppenhaltige Copolymerisat wird zusammen mit einen wasserlöslichen Amin eingesetzt. Besonders bevorzugt sind wasserlösliche Polyoxyalkylenamine der allgemeinen Formel. 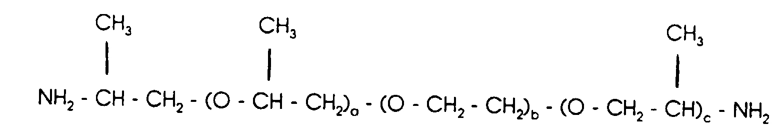 in der a + c größer als 2, b größer als 6 und kleiner als 60 ist.

Als Polyoxyalkylene mit einer Molekularmasse unter 1000 werden Polyethylenglykole, Polypropylenglykole, Polyoxypropylen/Polyoxyethylen-Copolymere, Monoethylenglykoldimethylether oder Polyethylenglykoldimethylether eingesetzt.

Als Polyole werden bevorzugt Glycerin oder dessen Derivate wie Diacetin, Glycerinaldehyd, Glycerinsäure, Glycerinsäuremethylester, α-Monoacetin oder α-Monobutirin.

Um eine erforderliche elektrische Leitfähigkeit der Haftklebemasse auf Acrylatbasis zu erreichen, wird dem Copolymerisat eine Elektrolytlösung (wässrige Lösung organischer oder anorganischer Salze) zugegeben. Besonders bevorzogt wird eine physiologisch unbedenkliche wässrige NaCl- oder KCl-Lösung.

Als geeignete Vernetzer, die mit den Carboxylgruppen eine intermolekulare Struktur ausbilden können, werden Metallchelate, Metallsäureester, Epoxid-, Aziridin- oder Melaminformaldehydharze empfohlen. Besonders geeignet sind bei Raumtemperatur reagierende Vernetzer wie beispielsweise Metallchelate.

Das carboxylgruppenhaltige Copolymerisat auf Acrylatbasis wird durch radikalische Polymerisation in Lösemittel in an sich bekannter Weise aus den benötigten Komponenten synthetisiert. Das erhaltene Polymerisat wird mit Isopropylalkohol stabilisiert und mit einem wasserlöslichen Amin, Polyoxyalkylen oder Polyol, wässriger Elektrolytlösung und einem Vernetzungsmittel gemischt. Die so erhaltene homogene Haftklebemasse wird auf eine siliconisierte Folie aufgetragen, im Trockenkanal bei 65°C getrocknet und dann durch Kaschieren auf eine entsprechende Unterlage zu einem elektrisch leitfähigen Haftklebeartikel verarbeitet.

Die Erfindung wird anhand der folgenden Beispiele näher erläutert.

### Beispiele 1-10

Die in den nachfolgenden Tabellen verwendeten Abkürzungen haben folgende Bedeutung:

### Abkürzungsverzeichnis

- 2-EHA -: 2-Ethylhexylacrylat
- IO -: Isooctylacrylat
- BA -: Butylacrylat
- HEA -: 2-Hydroxyethylacrylat
- HPA -: 2-Hydroxypropylacrylat
- HBA -: 4-Hydroxybutylacrylat
- AS -: Acrylsäure
- APS -: β-Acryloyloxypropionsäure
- VS-Na -: Natriumvinylsulfonat
- MAS-Na -: Natriummethallylsulfonat
- AMBS-NA -: Natriumsalz der 3-Acrylamido-3-methylbuttersäure
- AUS-Na-: Natriumsalz der 10-Acrylamido-undecansäure
- ED 600 -: Polyoxyalkylenamin mit einer Molekarmasse von 600
- ED 900 -: Polyoxyalkylenamin mit einer Molekularmasse von 900
- PEG 200 -: Polyethylenglykol mit einer Molekularmasse von 200
- PPG 405 -: Polypropylenglykol mit einer Molekularmasse von 405
- AIACA -: Aluminiumacetylacetonat
- ZrACA -: Zirkonacetylacetonat

Die aus den in Tabelle 1 aufgeführten Monomeren durch Lösungspolymerisation gebildeten Polymerisate weisen einen Feststoffgehalt von ca. 50 Gew.-% auf.

Nach Verdünnen der hergestellten Polymerisate mit Isopropylalkohol auf einen Feststoffgehalt von 33 Gew.-% wird die Polymerisatlösung mit den aus der Tabelle 2 ersichtlichen Komponenten wie wasserlösliches Amin, Polyoxyalkylen oder Polyol, Elektrolytlösung und Vernetzungsmittel gemischt. Die auf diese Weise erhaltenen Haftklebemassen wurden auf eine siliconisierte Polyesterfolie aufgetragen, 10 Minuten bei 65°C im Trockenkanal getrocknet und anschließend mit einer aluminisierten Polyesterfolie abgedeckt. Der Masseauftrag lag bei ca. 140 g/m²

Das fertige Produkt wurde 1 Woche bei Raumtemperatur konditioniert. Die elektrische Leitfähigkeit der transparenten Haftklebefilme wurde unter Verwendung eines Hochohm-Meßgeräts gemäß DIN 53 482 gemessen.

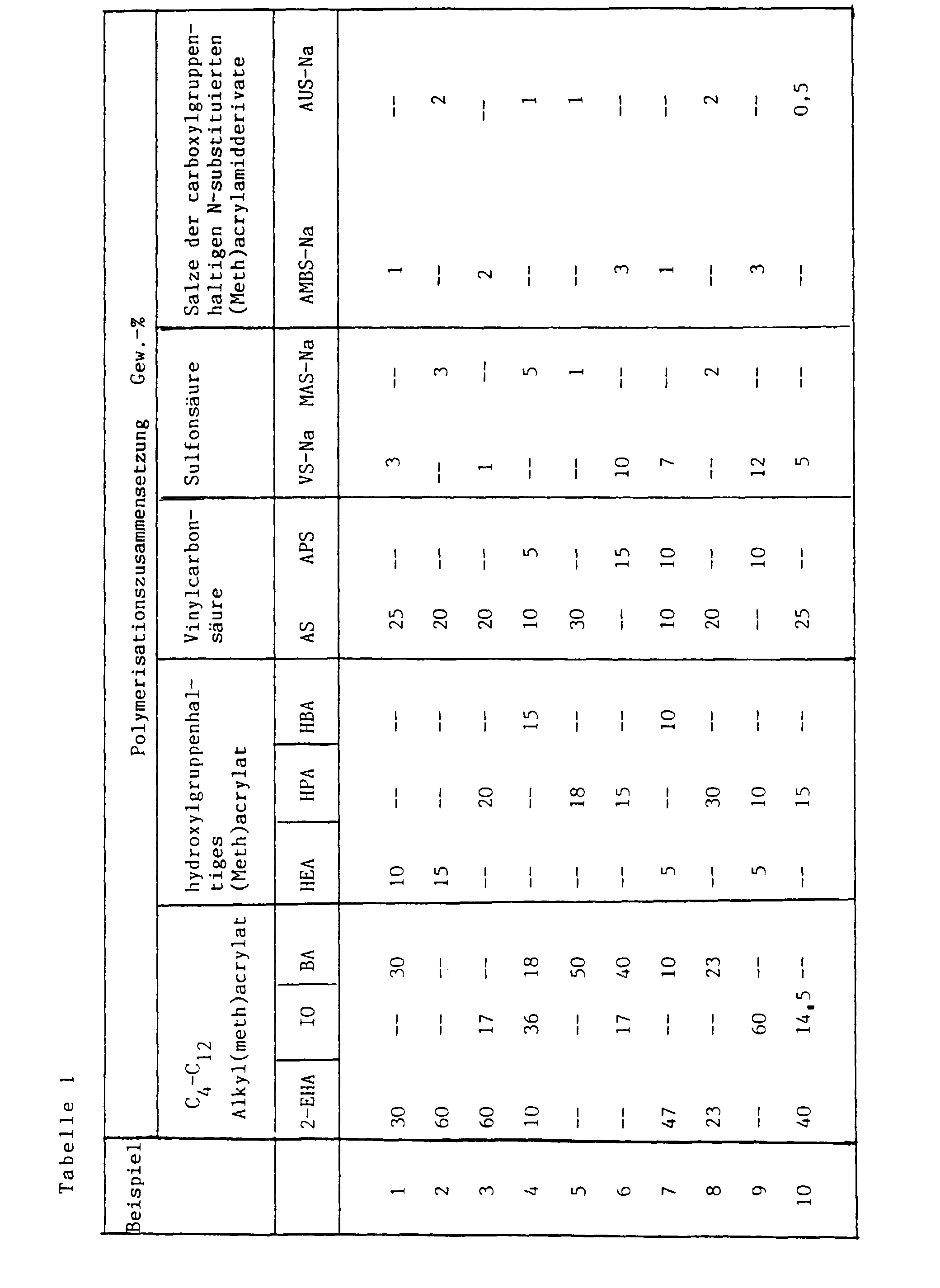

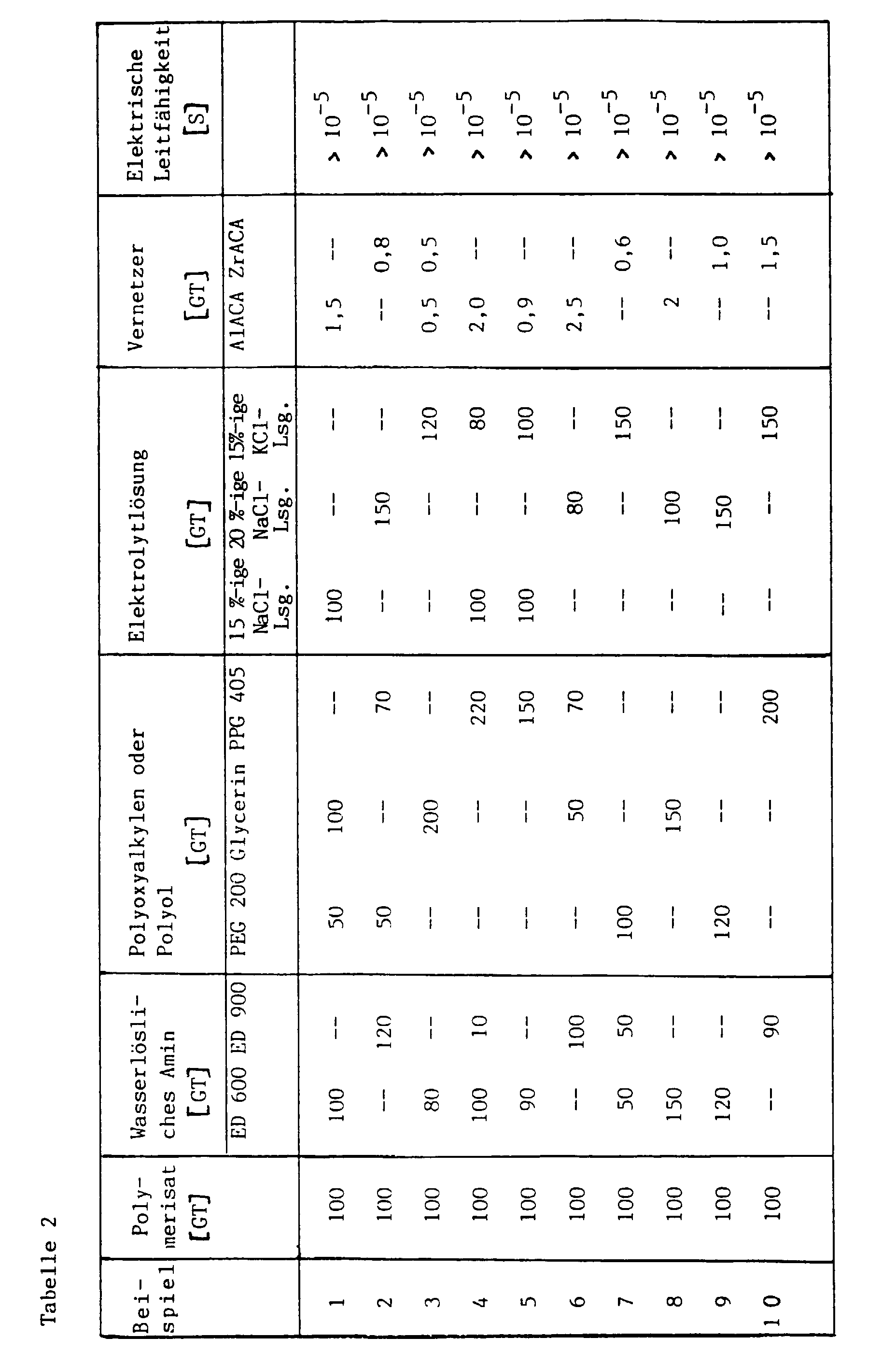

Aus Tabelle 2 ist zu ersehen, daß die ermittelte Leitfähigkeit durchweg oberhalb von 10⁻⁵ S lag, wodurch sich die Haftklebefilme in besonderer Weise zur Herstellung von biomedizinischen Elektroden eignen.

Beim Abziehen derartiger Haftklebefilme von der Haut verbleiben keine Kleberückstände auf der Haut, so daß eine Wiederverwendung der mit diesen elektrisch leitfähigen Haftklebefilmen ausgerüsteten biomedizinischen Elektroden ermöglicht wird.

### Beispiel 11

Zur Herstellung einer elektrisch leitfähigen Elektrode wurde der mit der nach dem Beispiel 1 synthetisierten Haftklebemasse hergestellte elektrisch leitfähige Haftklebefilm auf einen Polyethylenschaum übertragen. Zwischen den Polyethylenschaum und den zukaschierten elektrisch leitfähigen Haftklebefilm befand sich eine Reihe paralleler Elektrodenstreifen mit kurzem ca. 40 cm langen Anschlußkabel. Die Kontaktfläche betrug 40 x 40 mm.

## Patentansprüche

1. Elektrisch leitfähiger transparenter Haftklebefilm mit einer elektrischen Leitfähigkeit oberhalb von 10⁻⁵ S, bestehend aus
- 100 Gewichtsteilen eines carboxylgruppenhaltigen Copolymerisats auf Acrylatbasis,
- 50 bis 150 Gewichtsteilen eines wasserlöslichen Amins,
- 50 bis 250 Gewichtsteilen eines Polyoxyalkylens mit einer Molekularmasse unter 1000
und /oder eines Polyols
und/oder dessen Derivate,
- 50 bis 200 Gewichtsteilen einer Elektrolytlösung und
- 0,1 bis 6 Gewichtsteilen eines Vernetzungsmittels.

2. Haftklebefilm nach Anspruch 1, dadurch gekennzeichnet, daß das carboxylgruppenhaltige Copolymerisat besteht aus
a) 40 bis 80 Gew.-% Alkyl(meth)acrylate mit 4 bis 12 C-Atomen im Alkylrest,
b) 10 bis 30 Gew.-% hydroxylgruppenhaltiger (Meth)Acrylate.
c) 5 bis 30 Gew.-% einfach ungesättigter Carbonsäuren,
d) 0,5 bis 20 Gew.-% an Salzen von ungesättigten organischen Sulfonsäuren,
e) 0,1 bis 5 Gew.-% an Salzen von carboxylgruppenhaltigen N-substituierten (Meth)acrylamidderivaten.

3. Haftklebefilm nach Anspruch 1 oder 2, dadurch gekennzeichnet, daß das Alkyl(meth)acrylat mit 4 bis 12 C-Atomen im Alkylrest ein Butyl-, Pentyl-, Hexyl-, Heptyl-, Octyl-, 2-Ethylhexyl-, Isooctyl-, 2-Methylheptyl-, Nonyl-, Decyl- oder Dodecyl(meth)acrylat ist.

4. Haftklebefilm nach einem oder mehreren der Ansprüche 1 bis 3, dadurch gekennzeichnet, daß das hydroxylgruppenhaltige (Meth)acrylat ein 2-Hydroxyethyl(meth)acrylat, 2-Hydroxypropyl(meth)acrylat oder 4-Hydroxybutyl(meth)acrylat ist.

5. Haftklebefilm nach einen oder mehreren der Ansprüche 1 bis 4, dadurch gekennzeichnet, daß die einfach ungesättigte Carbonsäure aus (Meth)acrylsäure, β-Acryloyloxypropionsäure, Vinylessigsäure, Fumarsäure, Crotonsäure, Aconitsäure, Dimethylacrylsäure oder Itaconsäure ausgewählt ist.

6. Haftklebefilm nach einem oder mehreren der Ansprüche 1 bis 5, dadurch gekennzeichnet, daß die Salze der ungesättigten organischen Sulfonsäure Alkali - oder Ammoniumsalze sind.

7. Haftklebefilm nach einem oder mehreren der Ansprüche 1 bis 6, dadurch gekennzeichnet, daß die ungesättigte organische Sulfonsäure eine Vinylsulfonsäure, 2-Methylprop-1-en-3-sulfonsäure, Vinylbenzylsulfonsäure oder eine 2-Acrylamido-2-methylpropansulfonsäure ist.

8. Haftklebefilm nach einen oder mehreren der Ansprüche 1 bis 7, dadurch gekennzeichnet, daß die Salze des carboxylgruppenhaltigen N-substituierten (Meth)acrylamidderivates Alkali- oder Ammoniumsalze sind.

9. Haftklebefilm nach einem oder mehreren der Ansprüche 1 bis 8, dadurch gekennzeichnet, daß das carboxylgruppenhaltige N-substituierte (Meth)acrylamidderivat die allgemeine Formel 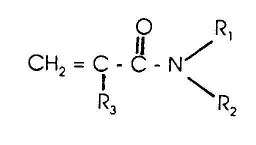 besitzt.
in der R₁ ein Wasserstoffatom, eine Alkyl-, Aryl-, Arylalkyl-, Alkylaryl-, Alkoxyalkyl-, Alkoxyaryl-, Acetylalkyl oder Acetylalkoxyalkylgruppe, R₂ eine Carboxylalkyl- oder Carboxylarylgruppe, R₃ ein Wasserstoffatom oder eine Methylgruppe sein können.

10. Haftklebefilm nach einem oder mehreren der Ansprüche 1 bis 9, dadurch gekennzeichnet, daß das wasserlösliche Amin ein Polyoxylalkylenamin, vorzugsweise der allgemeinen Formel 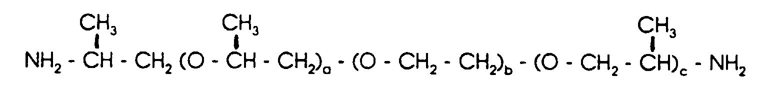 ist, in der a + c größer als 2, b größer als 6 und kleiner als 60 ist.

11. Haftklebefilm nach einen oder mehreren der Ansprüche 1 bis 10, dadurch gekennzeichnet, daß das Polyoxyalkylen mit einer Molekularmasse unter 1000 aus Polyethylenglykol, Polypropylenglykol,Polyoxypropylen/Polyoxyethylen-Copolymer, Monoethylenglykoldimethylether oder Polyethylenglykoldimethylether ausgewählt ist und das Polyol ein Glycerin oder dessen Derivate wie Diacetin, Glycerinaldehyd, Glycerinsäure, Glycerinsäuremethylester, α-Monoacetin oder α-Monobutirin ist.

12. Haftklebefilm nach einem oder mehreren der Ansprüche 1 bis 11, dadurch gekennzeichnet, daß die Elektrolytlösung eine physiologisch unbedenkliche wässrige organische oder anorganische Salzlösung ist .

13. Haftklebefilm nach einem oder mehreren der Ansprüche 1 bis 12, dadurch gekennzeichnet, daß der Vernetzer ein Metallchelat, ein Metallsäureester, ein Epoxid, ein Aziridin, ein Triazidin oder ein Melaminformaldehydharz ist.

14. Verfahren zur Herstellung eines Haftklebefilms gemäß einem oder mehreren der Ansprüche 1 bis 13, dadurch gekennzeichnet, daß
- 100 Gewichtsteile eines carboxylgruppenhaltigen Copolymerisat auf Acrylatbasis, welches auf an sich durch Polymerisation in Lösung erhalten und mit Isopropylalkohol stabilisiert wurde,
- 50 bis 150 Gewichtsteile eines wasserlöslichen Amins
- 50 bis 250 Gewichtsteile eines Polyoxyalkylens mit einer Molekularmasse unter 1000
und /oder eines Polyols
und /oder dessen Derivate,
- 50 bis 200 Gewichtsteile einer Elektrolytlösung und
- 0,1 bis 6 Gewichtsteile eines Vernetzungsmittels
gemischt und homogenisiert werden, daß die so erhaltene homogene Haftklebemasse auf eine gegebenenfalls oberflächenbehandelte Folie aufgetragen, getrocknet und auf die gewünschte Größe geschnitten wird.

15. Verwendung der elektrisch leitfähigen transparenten Haftklebefilme gemäß einem oder mehreren der Ansprüche 1 bis 14 zur Herstellung von biomedizinischen Elektroden.

## Claims

1. Electrically conductive, transparent, pressure-sensitive adhesive film with an electric conductivity above 10⁻⁵ S, consisting of
- 100 parts by weight of a carboxyl group-containing copolymer on an acrylate basis;
- 50 to 150 parts by weight of a water-soluble amine;
- 50 to 250 parts by weight of a polyoxyalkylene with
a molecular mass of below 1000
and/or a polyalcohol
and/or its derivatives;
- 50 to 200 parts by weight of a solution of electrolytes; and
- 0.1 to 6 parts by weight of a cross-linking agent.

2. Pressure sensitive adhesive film according to Claim 1, characterized in that the carboxyl group-containing copolymer consists of
a) 40 to 80 weight per cent of alkyl(meth)acrylates with 4 to 12 C-atoms in the alkyl radical,
b) 10 to 30 weight per cent of hydroxyl group-containing (meth)acrylates,
c) 5 to 30 weight per cent of monoethenoid carboxylic acids,
d) 0.5 to 20 weight per cent of salts of unsaturated organic sulfonic acids,
e) 0.1 to 5 weight per cent of salts of carboxyl group-containing, N-substituted (meth)acrylamide derivatives.

3. Pressure-sensitive adhesive film according to Claims 1 or 2, characterized in that the alkyl(meth)acrylate with 4 to 12 C-atoms in the alkyl radical is a butyl, pentyl, hexyl, heptyl, octyl, 2-ethylhexyl, isooctyl, 2-methylheptyl, nonyl, decyl or dodecyl(meth)acrylate.

4. Pressure-sensitive adhesive film according to one or more of the Claims 1 to 3, characterized in that the hydroxyl group-containing (meth)acrylate is a 2-hydroxyethyl(meth)acrylate, a 2-hydroxypropyl(meth)acrylate or a 4-hydroxybutyl(meth)acrylate.

5. Pressure-sensitive adhesive film according to one or more of the Claims 1 to 4, characterized in that the monoethenoid carboxylic acid is selected from (meth)acrylic acid, β-acryloyloxypropionic acid, vinyl acetic acid, fumaric acid, crotonic acid, aconitic acid, dimethyl acrylic acid or itaconic acid.

6. Pressure-sensitive adhesive film according to one or more of the Claims 1 to 5, characterized in that the salts of the unsaturated organic sulfonic acid are alkali or ammonium salts.

7. Pressure-sensitive adhesive film according to one or more of the Claims 1 to 6, characterized in that the unsaturated organic sulfonic acid is a vinyl sulphonic acid, a 2-methylprop-1-en-3-sulphonic acid, a vinylbenzyl sulfonic acid or a 2-acrylamido-2-methylpropane sulphonic acid.

8. Pressure-sensitive adhesive film according to one or more of the Claims 1 to 7, characterized in that the salts of the carboxyl group-containing N-substituted (meth)acrylamide derivatives are alkali or ammonium salts.

9. Pressure-sensitive adhesive film according to one or more of the Claims 1 to 8, characterized in that the carboxyl group-containing N-substituted (meth)acrylamide derivative has the general formula 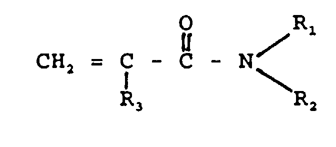 in which R₁ can be a hydrogen atom, an alkyl, aryl, arylalkyl, alkylaryl, alkoxyalkyl, alkoxyaryl, acetylalkyl or acetylalkoxyalkyl group; R₂ a carboxylalkyl or carboxylaryl group; and R₃ a hydrogen atom or a methyl group.

10. Pressure-sensitive adhesive film according to one or more of the Claims 1 to 9, characterized in that the water-soluble amine is a polyoxyalkylene amine, preferably with the general formula 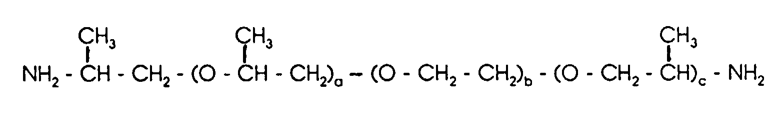 in which a + c is larger than 2 and b is larger than 6 and smaller than 60.

11. Pressure-sensitive adhesive film according to one or more of the Claims 1 to 10, characterized in that the polyoxyalkylene with a molecular mass below 1000 is selected from polyethylene glycol, polypropylene glycol, polyoxypropylene/polyoxyethylene copolymers, monoethylene glycol dimethyl ether or polyethylene glycol dimethyl ether, and that the polyalcohol is a glycerin or a derivative thereof, such as diacetin, glyceric aldehyde, glyceric acid, glyceric acid methylester, α-monoacetin or α-monobutyrin.

12. Pressure-sensitive adhesive film according to one or more of the Claims 1 to 11, characterized in that the solution of electrolytes is a physiologically safe, aqueous, organic or inorganic salt solution.

13. Pressure-sensitive adhesive film according to one or more of the Claims 1 to 12, characterized in that the cross-linking agent is a metallic chelate, metallic acid ester, an epoxide, an aziridine, a triazidine or a melamine formaldehyde resin.

14. Process for the production of a pressure-sensitive adhesive film according to one or more of the Claims 1 to 13, characterized in that
- 100 parts by weight of a carboxyl group-containing copolymer on an acrylate basis, obtained in solution in a known manner, by means of polymerization, and stabilized with isopropyl alcohol,
- 50 to 150 parts by weight of a water-soluble amine,
- 50 to 250 parts by weight of a solution of a polyoxyalkylene with a molecular mass of below 1000
and/or a polyalcohol
and/or its derivatives,
- 50 to 200 parts by weight of a solution of electrolytes and
- 0.1 to 6 parts by weight of a cross-linking agent
are mixed and homogenized; that the homogeneous pressure-sensitive adhesive mass thus obtained is applied to a sheet whose surface has been treated, if necessary, dried and cut to the required size.

15. Use of the electrically conductive, transparent, pressure-sensitive adhesive films according to one or more of the claims 1 to 14 for the production of biomedical electrodes.

## Revendications

1. Film autocollant transparent et conducteur de l'électricité, d'une conductivité électrique supérieure à 10⁻⁵ S, constitué de
- 100 parties en poids d'un copolymère à base d'acrylate contenant des radicaux carboxyle,
- 50 à 150 parties en poids d'une amine soluble dans l'eau,
- 50 à 250 parties en poids d'un polyalkylène d'une masse moléculaire inférieure à 1000
et/ou d'un polyol,
et/ou de ses dérivés,
- 50 à 200 parties en poids d'une solution d'électrolyte et
- 0,1 à 6 parties en poids d'un agent de réticulation.

2. Film autocollant suivant la revendication 1, caractérisé en ce que le copolymère contenant des groupes carboxyle se compose de
a) 40 à 80% en poids de (méth)acrylates d'alkyle dont le radical alkyle comporte de 4 à 12 atomes de carbone,
b) 10 à 30% en poids de (méth)acrylates contenant des radicaux hydroxyle,
c) 5 à 30% en poids d'acides carboxyliques mono-insaturés,
d) 0,5 à 20% en poids de sels d'acides sulfoniques organiques insaturés,
e) 0,1 à 5% en poids de sels de dérivés du (méth)acrylamide N-substitués et contenant des radicaux carboxyle.

3. Film autocollant suivant la revendication 1 ou 2, caractérisé en ce que le (méth)acrylate d'alkyle comportant de 4 à 12 atomes de carbone dans le reste alkyle est un (méth)acrylate de butyle, pentyle, hexyle, heptyle, octyle, 2-éthylhexyle, isooctyle, 2-méthylheptyle, nonyle, décyle et (méth)acrylate de dodécyle.

4. Film autocollant suivant l'une quelconque des revendications 1 à 3, caractérisé en ce que le (méth)acrylate contenant des radicaux hydroxyle est le (méth)acrylate de 2-hydroxyéthyle, le (méth)acrylate de 2-hydroxypropyle, ou le (méth)acrylate de 4-hydroxybutyle.

5. Film autocollant suivant l'une quelconque des revendications 1 à 4, caractérisé en ce que l'on choisit l'acide carboxylique monoinsaturé parmi l'acide (méth)acrylique, l'acide β-acryloyloxypropionique, l'acide vinylacétique, l'acide fumarique, l'acide crotonique, l'acide aconitique, l'acide diméthylacrylique, ou l'acide itaconique.

6. Film autocollant suivant l'une quelconque des revendications 1 à 5, caractérisé en ce que les sels de l'acide sulfonique organique insaturé sont des sels de métaux alcalins ou d'ammonium.

7. Film autocollant suivant l'une quelconque des revendications 1 à 6, caractérisé en ce que l'acide sulfonique organique insaturé est l'acide vinylsulfonique, l'acide 2-méthyl-prop-1-ène-3-sulfonique, l'acide vinylbenzylsulfonique, ou l'acide acrylamido-2-méthylpropanesulfonique.

8. Film autocollant suivant l'une quelconque des revendications 1 à 7, caractérisé en ce que les sels du dérivé du (méth)acrylamide N-substitués contenant des radicaux carboxyle sont des sels de métaux alcalins ou d'ammonium.

9. Film autocollant suivant l'une quelconque des revendications 1 à 8, caractérisé en ce que le dérivé du (méth)acrylamide N-substitué contenant des radicaux carboxyle répond à la formule générale 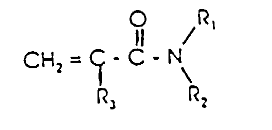 dans laquelle R₁ représente un atome d'hydrogène, un radical alkyle, aryle, arylalkyle, alkylaryle, alcoxyalkyle, alcoxyaryle, acétylalkyle ou acétylalcoxyalkyle, R₂ représente un groupe carboxyalkyle ou carboxyaryle, R₃ représente un atome d'hydrogène ou le radical méthyle.

10. Film autocollant suivant l'une quelconque des revendications 1 à 9, caractérisé en ce que l'amine soluble dans l'eau est une polyalkylèneamine répondant, de préférence, à la formule générale suivante 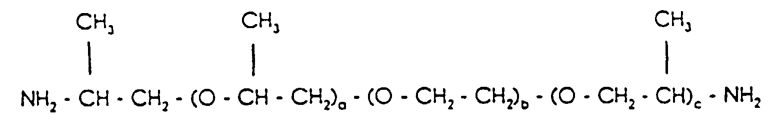 dans laquelle a + c est supérieur à 2, b est supérieur à 6 et inférieur à 60.

11. Film autocollant suivant l'une quelconque des revendications 1 à 10, caractérisé en ce que le polyalkylène d'une masse moléculaire inférieure à 1000 est choisi parmi le polyéthylèneglycol, le polypropylèneglycol, un copolymère de polyoxypropylène/polyoxyéthylène, l'éther diméthylique du monoéthylèneglycol, ou l'éther diméthylique du polyéthylèneglycol et en ce que le polyol est la glycérine ou ses dérivés, comme la diacétine, le glycérinaldéhyde, l'acide glycérique, le glycérate de méthyle, l'α-monoacétine, ou l'α-monobutyrine.

12. Film autocollant suivant l'une quelconque des revendications 1 à 11, caractérisé en ce que la solution d'électrolyte est une solution aqueuse de sel organique ou inorganique, physiologiquement irréprochable.

13. Film autocollant suivant l'une quelconque des revendications 1 à 12, caractérisé en ce que l'agent de réticulation est un chélate de métal, un ester d'acide de métal, un époxyde, une aziridine, une triazidine, ou une résine de mélamine-formaldéhyde.

14. Procédé de fabrication d'un film autocollant suivant une ou plusieurs des revendications 1 à 13, caractérisé en ce que l'on mélange et homogénéise
- 100 parties en poids d'un copolymère à base d'acrylate contenant des radicaux carboxyle, qui a été obtenu par polymérisation en solution et stabilisé avec de l'alcool isopropylique,
- 50 à 150 parties en poids d'une amine soluble dans l'eau,
- 50 à 250 parties en poids d'un polyoxyalkylène d'une masse moléculaire inférieure à 1000
et/ou d'un polyol,
et/ou de ses dérivés,
- 50 à 250 parties en poids d'une solution d' électrolyte et
- 0,1 à 6 parties en poids d'un agent de réticulation,
en ce que l'on applique la masse de colle de contact homogène ainsi obtenue sur une feuille éventuellement traitée en surface, on la sèche et on la découpe à la taille voulue.

15. Utilisation des films autocollants transparents et conducteurs de l'électricité suivant une ou plusieurs des revendications 1 à 14 en vue de la fabrication d' électrodes biomédicales.
